# EUROPÄISCHE PATENTANMELDUNG

(11) **EP 0 620 211 A1**
(43) Veröffentlichungstag der Anmeldung: **19.10.1994**
(21) Anmeldenummer: 94102103.2
(22) Anmeldetag: 11.02.1994
(51) Int. Cl.: C07C 251/08, C08G 18/32, C08G 18/10, C07D 251/34

(54) **Azomethine, ein Verfahren zu ihrer Herstellung, sowie deren Verwendung zur Herstellung azomethinhaltigen Polyurethanen**

(30) Priorität: 10.04.1993 DE 4311901
(71) Anmelder: HÜLS AKTIENGESELLSCHAFT, D-45764 Marl (DE)
(72) Erfinder: Lomölder, Rainer, Dr., D-48153 Münster (DE); Paulen, Wilfried, Dr., D-45657 Recklinghausen (DE); Schmitt, Felix, Dr., D-45701 Herten (DE); Wolf, Elmar, Dr., D-45661 Recklinghausen (DE)

(57) **Zusammenfassung**

Die Erfindung betrifft neue Azomethine (Schiffsche Basen) folgender Zusammensetzung: 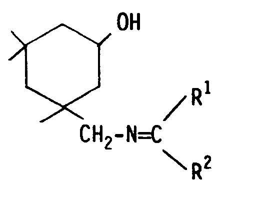
wobei:
- R¹:: H, C₁₋₁₄-Alkyl-Rest, der gegebenenfalls verzweigt ist, und
- R²:: C₁₋₁₄-Alkyl-Rest, der gegebenenfalls verzweigt ist und alkylsubstituierter Phenyl-Rest
bedeuten.

Sie betrifft ferner ein Verfahren zur Herstellung der Azomethine, wobei 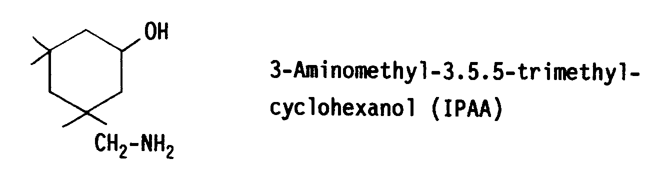
mit den entsprechenden Carbonylverbindungen 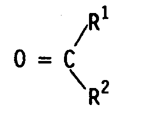
umgesetzt wird.

Weiterhin betrifft die Erfindung die Verwendung der Azomethine zur Herstellung von bestimmten Polyazomethinen (Poly-Schiffschen Basen), wobei die Azomethine mit Diisocyanaten oder deren Addukten mit Polyolen oder deren Trimerisate umgesetzt werden.

## Beschreibung

Vorliegende Erfindung betrifft neue Azomethine, ein Verfahren zu ihrer Herstellung sowie deren Verwendung.

Azomethine, bekannter unter der Bezeichnung Schiffsche Basen, werden in der Regel durch Kondensation primärer Amine und Carbonylverbindungen hergestellt: 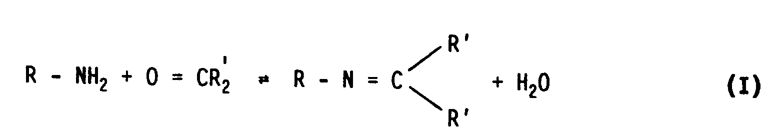
Wird das H₂O aus dem Gleichgewicht entfernt, kann das Azomethin in nahezu quantitativer Ausbeute erhalten werden. Primäre 2-Hydroxyamine gehen mit Carbonylverbindungen in ein Gemisch von Schiffschen Basen und Oxazolidinen über (A. Paquin, B. 82, 316 (1949)), wobei die beiden Reaktionsprodukte miteinander im Gleichgewicht stehen: 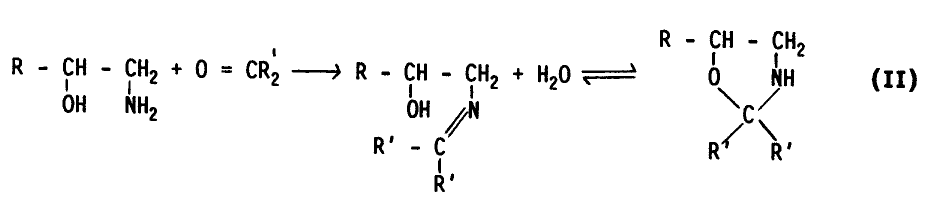
Analog reagieren 3- bzw. 4-Hydroxyamine mit Carbonylverbindungen zu 6-bzw. 7gliedrigen Ringsystemen.

Aus einem 4-Hydroxyamin und einer Carbonylverbindung - Aldehyd oder Keton- kann die Schiffsche Base nach Gleichung I nur im Gemisch mit einem 7-Ring-N,O-Heterocyclus hergestellt werden. Eine Schiffsche Base aus einem 4-Hydroxyamin wäre zur Herstellung von Poly-Schiffschen Basen von großem Interesse, da durch Hydrolyse dann auf einfache Weise Polyamine erhalten werden, die auf andere Weise nicht bzw. nur sehr schwierig zugänglich sind.

So war es Aufgabe der Erfindung, nach 4-Hydroxyaminen zu suchen, die mit Carbonylverbindungen auf einfache Weise und ohne Nebenproduktbildung zu Azomethinen führen, deren OH-Gruppen in δ-Stellung zum N der Azomethingruppe steht.

Gelöst wurde diese Aufgabe durch Einsatz von Hydroxyaminen der allgemeinen Formel: 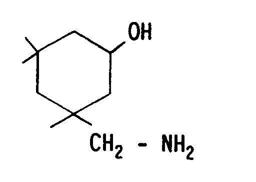
Gegenstand der vorliegenden Erfindung sind daher Azomethine (Schiffsche Basen) der allgemeinen Zusammensetzung: 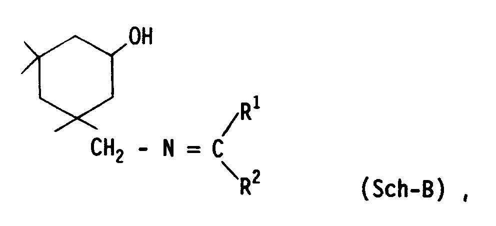
wobei:
- R¹:: H, C₁₋₁₄-Alkyl-Rest, der gegebenenfalls verzweigt ist, und
- R²:: C₁₋₁₄-Alkyl-Rest, der gegebenenfalls verzweigt ist und alkylsubstituierter Phenyl-Rest
bedeuten.

Bei den erfindungsgemäßen Verbindungen handelt es sich um niedrigviskose Produkte mit einer Farbzahl nach Gardner < 1, und einem Amingehalt von 2 bis 5 mmol/g und einer OH-Zahl (mg KOH/g) von 100 bis 280.

Ein weiterer Gegenstand der vorliegenden Erfindung ist ein Verfahren zur Herstellung der Azomethine nach Anspruch 1, welches dadurch gekennzeichnet ist, daß folgender Aminoalkohol: 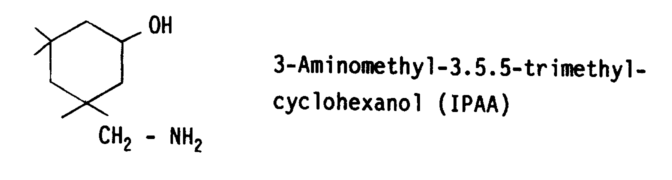
mit folgenden Carbonylverbindungen: 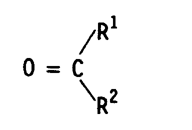
wobei:
- R¹:: H; C₁₋₁₄-Alkyl-Rest, der gegebenenfalls verzweigt ist
- R²:: C₁₋₁₄-Alkyl-Rest, der gegebenenfalls verzweigt ist und alkylsubstituierter Phenylrest
bedeuten,
im Molverhältnis 1 : 1 - 1,3 bei Temperaturen von 60 bis 140 °C in bekannter Weise umgesetzt wird.

Die Herstellung kann sowohl in Lösung als auch lösungsmittelfrei erfolgen. Wird die Reaktion in Lösung durchgeführt, arbeitet man bevorzugt mit Toluol oder Xylol als (inerte) Lösungsmittel. Durch Zusatz von 0,01 - 0,1 Gew.-% an Säure, wie z. B. H₃PO₄, kann die Azomethinbildung beschleunigt werden. Dies ist dann zweckmäßig, wenn es sich um träge Reaktionskomponenten, wie dies bei sterisch gehinderten Aminen und Ketonen der Fall ist, handelt. IPAA und Toluol werden bei Raumtemperatur gemischt und mit der Carbonylverbindung im molaren Verhältnis versetzt. Die Konzentration des Toluols beträgt ca. 50 bis 80 Gew.-%. Die Lösung wird dann langsam bis zum Rückfluß erhitzt und so lange am Wasserabscheider weiter erhitzt, bis die berechnete H₂O-Menge abdestilliert ist. Danach wird das Toluol im Vakuum abdestilliert. Die Konzentration der so hergestellten Schiffschen Basen beträgt ≧ 99 % (Flächen-% im Gaschromatogramm). In der Regel ist diese Reinheit für die Weiterreaktion der Schiffschen Basen mit NCO-Präpolymeren zur Bildung von Poly-Schiffschen Basen ausreichend.

Bei der Herstellung in Substanz werden IPAA und die Carbonylkomponente im molaren Verhältnis bei Raumtemperatur gemischt und langsam bis zum Rückfluß erhitzt und so lange weiter erhitzt, bis die berechnete H₂O-Menge abdestilliert ist. Es hat sich als sehr vorteilhaft erwiesen, die Carbonylkomponente im Überschuß von 10 bis 30 % zuzugeben. Nach der destillativen Abtrennung des Reaktionswassers und der überschüssigen Carbonylkomponente wird kurzzeitig Vakuum angelegt. Das so hergestellte erfindungsgemäße Verfahrensprodukt hat eine Reinheit von ≧ 99 % und braucht in der Regel nicht mehr weiter gereinigt zu werden.

Ein weiterer Gegenstand der vorliegenden Erfindung betrifft die Verwendung der erfindungsgemäßen Azomethine zur Herstellung von Polyazomethinen (Poly-Schiffschen Basen) folgender Zusammensetzung: 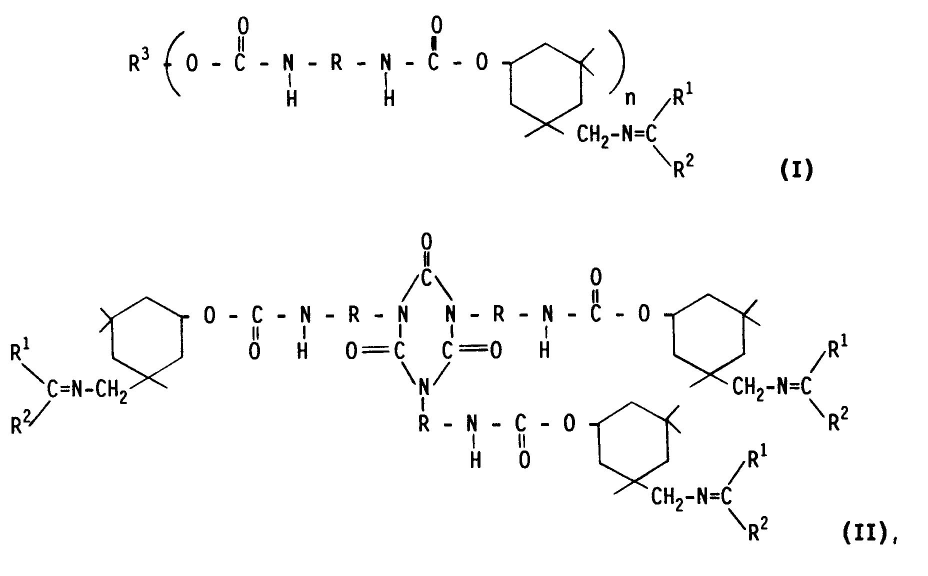
wobei 1 ≦ n ≦ 6 ist,
- R:: den KW-Rest eines (cyclo)aliphatischen oder aromatischen Diisocyanates mit 6 - 20 C-Atomen oder deren Trimerisaten,
- R³:: wenn 2 ≦ n ≦ 6, einen n-wertigen organischen Rest, wie er durch Entfernung von n OH-Gruppen aus einer gegebenenfalls Ethersauerstoffatome enthaltenden Polyhydroxylverbindung oder einer hydroxylhaltigen Polyesterverbindung mit einem durchschnittlichen Molgewicht zwischen 40 und 5 000 entsteht, wenn n = 1, 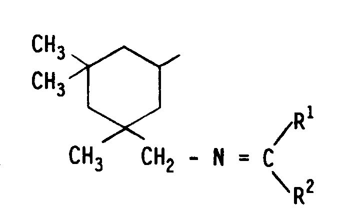 darstellen, und R¹ und R² die Bedeutung wie in Anspruch 1 besitzen.

Die Herstellung der erfindungsgemäßen Polyazomethine erfolgt durch Umsetzung von Diisocyanaten bzw. deren Addukten folgender Zusammensetzung: 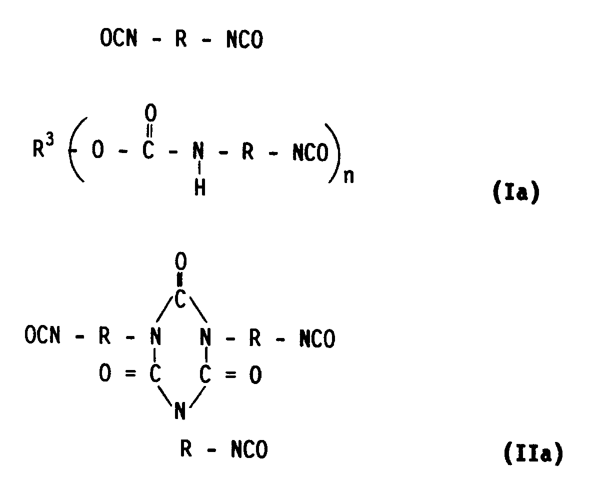
mit den erfindungsgemäßen Azomethinen der allgemeinen Zusammensetzung: 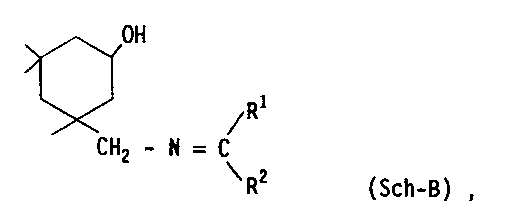
wobei R, R¹, R² und R³ die vorstehend genannte Bedeutung besitzen
im NCO : OH-Äquivalzenzverhältnis von 1 : 1.

Die Umsetzung erfolgt je nach Aggregatzustand des eingesetzten Diisocyanats bzw. NCO-Präpolymeren in Substanz oder in aprotischen Lösungsmitteln bei Temperaturen von 20 bis 80 °C.

Bei niedrigviskosen NCO-Präpolymeren kann auf Lösungsmittel verzichtet werden; bei hochviskosen und festen NCO-Präpolymeren muß dagegen in Lösung gearbeitet werden. Als Lösungsmittel kommen im Prinzip alle in Frage, die keine gegenüber NCO-Gruppen reaktiven funktionellen Gruppen enthalten. Besonders geeignet haben sich Ketone, wie z. B. Aceton, Methylethylketon und aromatische Kohlenwasserstoffe, wie z. B. Toluol, erwiesen.

Die für das erfindungsgemäße Verfahren eingesetzten Isocyanatpräpolymeren (Ia; IIa) werden nach an sich bekannten Methoden durch Umsetzung von Polyhydroxylverbindungen mit Diisocyanaten bzw. durch Trimerisierung von Diisocyanaten hergestellt.

Als Polyhydroxylverbindungen kommen Polyole mit einem Molgewicht von 60 bis 600, wie z. B. Ethylenglykol, Hexandiol, Di- und Triethylenglykol, Neopentylglykol, Trimethylolpropan, Octandecandiol, C₃₆-Diol, in Frage. Bevorzugt eignen sich Polyetherpolyole mit einem Molgewicht von 200 - 5 000, mit 2 - 5, vorzugsweise 2 -3 Hydroxylgruppen. Die erfindungsgemäß in Frage kommenden OH-Gruppen aufweisenden Polyether sind solche der an sich bekannten Art und werden z. B. durch Polymerisation von Epoxiden, wie Ethylenoxid, Propylenoxid, Tetrahydrofuran, Styroloxid mit sich selbst, z. B. in Gegenwart von Lewis-Säuren, wie z. B. BF₃, oder durch Anlagerung dieser Epoxide, gegebenenfalls im Gemisch oder nacheinander an Starterkomponenten mit reaktionsfähigen Wasserstoffatomen, wie H₂O, Alkohole und Amine, hergestellt. Weiterhin werden OH-Gruppen enthaltende Polybutadiene für die Isocyanatpräpolymeren eingesetzt sowie hydroxylgruppenhaltige Polyester und Polyketone.

Als Ausgangskomponente zur Herstellung der für das erfindungsgemäße Verfahren benötigten Isocyanatpräpolymeren (Ia, IIa) kommen (cyclo)aliphatische, araliphatische, aromatische Diisocyanate in Betracht, wie sie z. B. von W. Siefken in Justus Liebigs Annalen der Chemie, Bd. 562, S. 75-136, beschrieben werden, beispielsweise 1.6-Hexamethylendiisocyanat, 2-Methylpentamethylendiisocyanat-1.5, 1.12-Dodecandiisocyanat, Isophorondiisocyanat, Tetramethylxylylendiisocyanat, 2.4- und 2.6-Toluylendiisocyanat und Diphenylmethan-2.4'- und/oder 4.4'-diisocyanat.

Die Isocyanatpräpolymeren werden in der Regel so hergestellt, daß auf 1 OH-Äquivalent des Polyols 2 NCO-Äquivalente des Diisocyanats in bekannter Weise miteinander umgesetzt werden. Die so hergestellten Isocyanatpräpolymeren enthalten je nach Molgewicht noch ca. 2 - 8 % freies Diisocyanat. In manchen Fällen hat es sich als zweckmäßig erwiesen, Isocyanatpräpolymere mit einem Diisocyanatgehalt < 0,5 % für das erfindungsgemäße Verfahren einzusetzen. Solche "monomerarmen" Isocyanatpräpolymeren werden so hergestellt, daß in einer 1. Stufe das Diisocyanat in großem Überschuß mit dem Polyol zur Reaktion gebracht, und in einer 2. Stufe das nicht umgesetzte Diisocyanat durch Dünnschichtdestillation vom Reaktionsprodukt abgetrennt wird. Die so hergestellten Isocyanatpräpolymeren enthalten, unabhängig von ihrem Molgewicht, < 0,5 % Diisocyanat.

Die für das erfindungsgemäße Verfahren eingesetzten Isocyanatpräpolymeren II a werden in bekannter Weise durch Trimerisierung der bereits für die Herstellung der Isocyanatpräpolymeren I a aufgezählten Diisocyanate hergestellt. Als Trimerisierungskatalysatoren haben sich die in der DE-PS 26 44 684 und DE-PS 29 16 201 beschriebenen als vorteilhaft erwiesen.

Als Reaktionskomponente für die Diisocyanate und die Isocyanatpräpolymeren (I a, II a) werden nach den erfindungsgemäßen Verfahren die erfindungsgemäßen Azomethine (Sch-B) eingesetzt.

Die erfindungsgemäßen Verfahrensprodukte eignen sich in hervorragender Weise zur einfachen Herstellung von Polyaminen, die auf andere Weise nicht oder nur schwierig hergestellt werden können.

### Experimenteller Teil

### A. Herstellung der erfindungsgemäßen Azomethine (Mono-Schiffschen) Basen

### Beispiel 1

342 Gew. -T. IPAA wurden mit 260 Gew. -T. Methyl-iso-butylketon (MIBK) bei Raumtemperatur gemischt und langsam bis zum Rückfluß am Wasserabscheider erhitzt und anschließend so lange bei dieser Temperatur (ca. 130 °C) weiter erhitzt, bis 36 Gew.-T. H₂O und das überschüssige MIBK abdestilliert wurden. Danach wurde das Reaktionsprodukt im Wasserstrahlvakuum andestilliert (2 % Destillat). Das so hergestellte Reaktionsprodukt konnte ohne weitere Rektifizierung zur Herstellung von Poly-Schiffschen Basen eingesetzt werden. Der Amin-Gehalt des Reaktionsproduktes betrug 3,89 mmol NH₂/g; seine Viskosität (23 °C) lag bei 2 610 mPa·s.

### Beispiel 2

342 Gew. -T. IPAA wurden mit 180 Gew.-T. Methylethylketon analog Beispiel 1 umgesetzt.
Das Reaktionsprodukt hatte einen basischen Amingehalt von 4,3 mmol NH₂/g und eine Viskosität bei 23 °C von 2 130 mPa·s.

### Beispiel 3

342 Gew.-T. IPAA wurden mit 300 Gew.-T. Diisobutylketon analog Beispiel 1 umgesetzt.
Das Reaktionsprodukt hatte einen basischen Amingehalt von 3,25 mmol NH₂/g und eine Viskosität bei 23 °C von 15 300 mPa·s.

### Beispiel 4

342 Gew.-T. IPAA und 180 Gew.-T. Isobutyraldehyd wurden mit 500 Gew.-T. Toluol bei Raumtemperatur gemischt und langsam am Wasserabscheider bis zum Rückfluß erhitzt und so lange bei dieser Temperatur gehalten, bis 36 Gew.-T. H₂O abdestilliert waren. Die Hauptmenge Toluol (und der nicht umgesetzte Isobutyraldehyd) wurde bei Normaldruck abdestilliert. Zur Beseitigung der Restmenge Toluol wurde das Reaktionsprodukt noch kurzzeitig im Wasserstrahlvakuum weiter erhitzt.

Das so hergestellte Azomethin hatte einen Reinheitsgrad von > 99 % (Flächen-% im Gaschromatogramm), einen basischen Amingehalt von 4,3 mmol NH₂/g und eine Viskosität bei 23 °C von 1 320 mPa·s.

### Beispiel 5

342 Gew. -T. IPAA und 285 Gew. -T. 3.5.5-Trimethylcyclohexanon wurden mit 500 Gew.-T. Toluol analog Beispiel 4 umgesetzt.
Das Reaktionsprodukt hatte einen basischen Amingehalt von 3,39 mmol NH₂/g und eine Viskosität von 31 600 mPa·s.

### B. Herstellung der erfindungsgemäßen Polyazomethine (Poly-Schiffschen Basen)

### Beispiel 1

253 Gew.-T. der Schiffschen Base Beispiel A. 1 und 560 Gew.-T. eines NCO-Präpolymeren, das nach bekanntem Verfahren aus 444 Gew.-T. IPDI und 650 Gew.-T. eines Polytetrahydrofurandiols mit durchschnittlichem Molgewicht von 650 hergestellt wurde, wurden bei 50 °C so lange zusammen erhitzt, bis kein NCO mehr nachgewiesen werden konnte (ca. 20 h).
Das Reaktionsprodukt hatte einen basischen Amingehalt von 1,2 mmol NH₂/g und eine Viskosität bei 23 °C von 750·10³ mPa·s.

### Beispiel 2

225 Gew.-T. der Schiffschen Base Beispiel A. 2 und 737 Gew.-T. eines NCO-Präpolymeren, das nach bekanntem Verfahren aus 444 Gew.-T. IPDI und 1000 Gew.-T. eines Polytetrahydrofurandiols mit durchschnittlichem Molgewicht von 1 000 hergestellt wurde, wurden analog Beispiel 1 umgesetzt.
Das Reaktionsprodukt hatte einen basischen Amingehalt von 1,03 mmol/NH₂/g und eine Viskosität bei 23 °C von 810·10³ mPa·s.

### Beispiel 3

295 Gew.-T. der Schiffschen Base Beispiel A. 3 und 1 313 Gew.-T. eines Isocyanatpräpolymeren, das nach bekanntem Verfahren aus 444 Gew.-T. IPDI und 2 000 Gew.-T. eines bifunktionellen Polypropylenglykols mit einem durchschnittlichen Molgewicht von 2 000 hergestellt wurde, wurden analog Beispiel 1 umgesetzt.

Das Reaktionsprodukt hatte einen basischen Amingehalt von 0,6 mmol NH₂/g und eine Viskosität bei 23 °C von 67·10³ mPa·s.

### Beispiel 4

244 Gew.-T. trimeres IPDI mit 17,2 % NCO (VESTANAT T 1890, Verkaufsprodukt der Hüls AG) wurden in 300 Gew.-T. Aceton gelöst und mit 253 Gew.-T. der Schiffschen Base A. 1 bei 50 °C so lange erhitzt, bis der NCO-Gehalt < 0,1 % betrug. Anschließend wurde das Aceton unter Normaldruck abdestilliert. Zur vollständigen Entfernung des Acetons wurde das Reaktionsprodukt noch im Wasserstrahlvakuum bei 120 °C weiter erhitzt.
Das Reaktionsprodukt hatte einen basischen Amingehalt von 1,2 mmol NH₂/g und einen Schmelzbereich von 165 - 169 °C.

## Patentansprüche

1. Neue Azomethine (Schiffsche Basen) folgender Zusammensetzung: 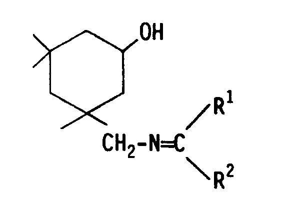 wobei:
R¹: H, C₁₋₁₄-Alkyl-Rest, der gegebenenfalls verzweigt ist, und
R²: C₁₋₁₄-Alkyl-Rest, der gegebenenfalls verzweigt ist und alkylsubstituierter Phenyl-Rest
bedeuten.

2. Verfahren zur Herstellung der Azomethine gemäß Anspruch 1,
dadurch gekennzeichnet,
daß folgender Aminoalkohol: 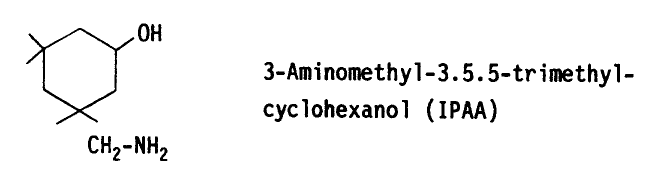 mit folgenden Carbonylverbindungen: 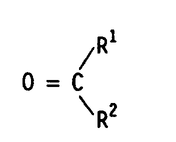 wobei:
R¹: H; C₁₋₁₄-Alkyl-Rest, der gegebenenfalls verzweigt ist
R²: C₁₋₁₄-Alkyl-Rest, der gegebenenfalls verzweigt ist und alkylsubstituierter Phenylrest
bedeuten,
im Molverhältnis 1 : 1 - 1,3 bei Temperaturen von 60 bis 140 °C in bekannter Weise umgesetzt wird.

3. Verwendung der Azomethine nach Anspruch 1 zur Herstellung von Polyazomethinen (Poly-Schiffschen Basen) folgender Zusammensetzung: 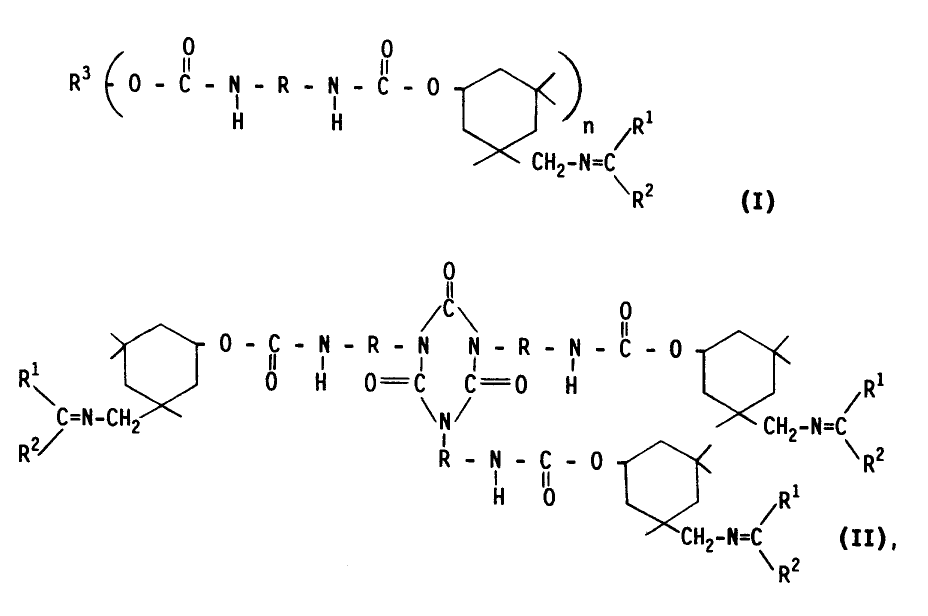 wobei 1 ≦ n ≦ 6 ist,
R: den KW-Rest eines (cyclo)aliphatischen oder aromatischen Diisocyanates mit 6 - 20 C-Atomen oder deren Trimerisaten,
R³: wenn 2 ≦ n ≦ 6, einen n-wertigen organischen Rest, wie er durch Entfernung von n OH-Gruppen aus einer gegebenenfalls Ethersauerstoffatome enthaltenden Polyhydroxylverbindung oder einer hydroxylhaltigen Polyesterverbindung mit einem durchschnittlichen Molgewicht zwischen 40 und 5 000 entsteht,
wenn n = 1, 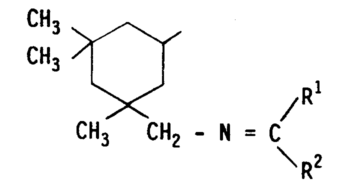 darstellen und R¹ und R² die Bedeutung wie in Anspruch 1 besitzen.

4. Verwendung der Azomethine nach Anspruch 1 zur Herstellung von Polyazomethinen (Poly-Schiffschen Basen) nach Anspruch 3,
dadurch gekennzeichnet,
daß die Azomethine gemäß Anspruch 1 mit Diisocyanaten oder deren Addukten mit Polyolen (I a) oder deren Trimerisate (II a) 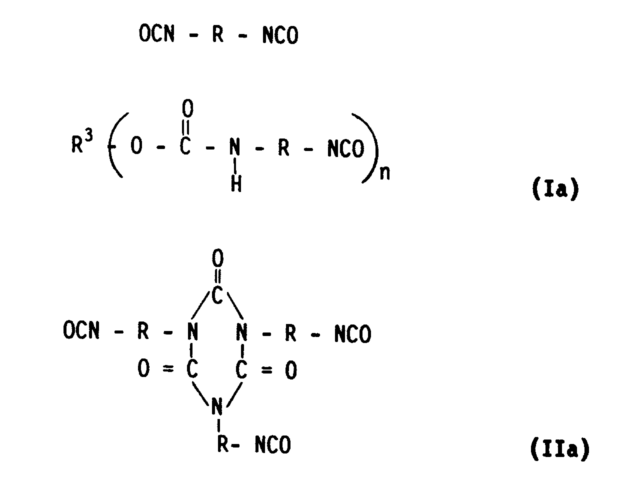 im OH : NCO-Äquivalenzverhältnis von 1 : 1 in bekannter Weise umgesetzt werden, und wobei n, R, R₁, R₂ und R₃ die Bedeutung wie in Anspruch 3 besitzen.
